(19) Europäisches Patentamt
European Patent Office
Office européen des brevets

(11) Publication number: **0 144 204**
**B1**

(12) **EUROPEAN PATENT SPECIFICATION**

(45) Date of publication of patent specification: **01.02.89**

(51) Int. Cl.$^4$: **C 07 C 102/04,** C 07 C 103/76

(21) Application number: **84308165.4**

(22) Date of filing: **26.11.84**

# (54) Process for preparing salicylamide compounds.

(30) Priority: **05.12.83 US 558066**

(43) Date of publication of application:
**12.06.85 Bulletin 85/24**

(45) Publication of the grant of the patent:
**01.02.89 Bulletin 89/05**

(84) Designated Contracting States:
**AT BE CH DE FR GB IT LI NL SE**

(56) References cited:
**EP-A-0 038 191**
**EP-A-0 038 192**

**ORGANIC SYNTHESES, vol. 26, 1946, pages 92-94; C.F.H. ALLEN et al.: "Salicyl-o-toluide"**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE**

(72) Inventor: **Koermer, Gerald Stephen**
**496, Mountain Avenue**
**Springfield New Jersey (US)**
Inventor: **Guttierez, Eddie Nelson**
**216 Bellemeade Avenue**
**Fort Lee New Jersey (US)**
Inventor: **Prorok, Maryfrances**
**444 Knickerbocker Road**
**Tenafly New Jersey (US)**

(74) Representative: **Doucy, Robert Henry et al**
**Unilever PLC Patents Division P.O. Box 68**
**Unilever House**
**London EC4P 4BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 144 204 B1**

## Description

This invention relates to a new and improved process for the preparation of salicylamide compounds.

Salicylamide compounds, especially those bearing n-alkyl or n-alkanoyl substituents in the benzene ring of the salicylic acid portion thereof, are described in US Patents Nos. 4 358 443 and 4 287 191 which disclose the use of such compounds as bactericides and as anti-dental plaque agents.

The synthesis of a typical unsubstituted salicylamide is conventionally conducted by the reaction of salicylic acid and aniline in the presence of phosphorus trichloride as condensing agent. US Patents Nos. 2 763 683; 3 221 051; 3 221 052 and 3 231 611 describe various processes for the preparation and purification of salicylanilide. However, while the processes described therein are presumably quite effective for the preparation and purification of the parent salicylanilide, they are not practical for the preparation of salicylanilides substituted in the salicyl and/or aniline portions of the molecule or other salicylamide compounds wherein the aniline portion of the molecule is wholly replaced by a substituted or unsubstituted heterocyclic amine. There is another method for the preparation of salicylamides known as the salol reaction. Salol is a common name for phenyl salicylate and the salol process involves the formation of amides of salicylic acid by the heating of phenyl salicylate with an amine which may be an aniline or a heterocyclic amine. The earliest report relative to the salol process appears to be by M. Schopff, *Ber.25,* 2740 (1892). A concise description of the salol process in the English language is contained in the Merck Index, 8th Edition, page 1211 together with a listing of various other publications in which the salol reaction has been mentioned or discussed.

According to the salol process, a given quantity of phenyl salicylate is heated with at least a stoichiometric corresponding quantity of aniline, usually in a high boiling aromatic diluent at temperature approaching and even exceeding 200°C for several hours. The resulting reaction product is thereafter allowed to crystallise, washed with ligroin and optionally recrystallised from ethanol. The aforementioned process has been described with reference to the preparation of both the parent salicylanilide as well as several salicylanilides where the substitution has been in the benzene ring of the aniline portion alone.

According to a report by C. F. H. Allen et al in "Organic Syntheses, Collective Volume III", 765 (1955), when the parent salicylanilide compound is synthesised the yield is only about 70% and moreover, the resulting product has a persistent pink colour which is not easily removed. While Allen, et al do report higher yields in the syntheses of other salicylamides, it is interesting to note that the only variation from the basic salol process synthesis of salicylanilide suggested therein is one involving the use of different anilines and heterocyclic amines. There is no suggestion made therein with respect to the usefulness or otherwise of the salol process in those cases where the phenyl salicylate reactant itself incorporates a substituent or substituents upon its salicylic acid benzene ring.

Due consideration of mechanistic concepts enables one to conclude that substitution of the benzene ring of the salicylic acid portion of the phenyl salicylate molecule with an alkyl and especially an alkanoyl group would lead to considerably more complexity in the reaction pathway as a result of the complexation of the aniline or other amine with the alkyl or alkanoyl substituent group. The result would be lower yields of the desired product, increased side reactions and more difficulty in purifying the desired salicylamide product.

It is an object of this invention to provide a new and improved process for the preparation in high yield and with relatively high reaction rates of salicylamides of the formula:—

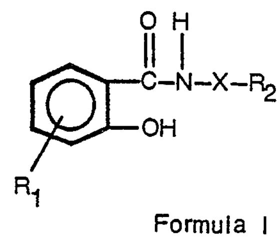

Formula I

where

X is a benzene ring or a heterocyclic aromatic group

$R_1$ is a hydrogen atom or a substituent group which is $—COC_nH_{2n+1}$ or $—C_nH_{2n+1}$ where n is an integer of from 1 to 15, and

$R_2$ is a hydrogen atom or a substituent group which is CN, $NO_2$, F, Cl, Br, I, $CF_3$, $CCl_3$, $CBr_3$, $CI_3$, $COC_nH_{2n+1}$ or $C_nH_{2n+1}$.

According to the present invention there is provided a process for the preparation of salicylamides of Formula I, which process comprises reacting together one mole part of a phenyl salicylate ester optionally bearing an $R_1$ substituent on the benzene ring of the salicylic acid portion thereof with about one to about two mole parts of an amine $NH_2—X—R_2$ and with about 0.01 to about 0.25, preferably about 0.05 to about 0.10, mole part of a Lowry-Bronsted acid catalyst at a temperature of above about 150°C, preferably between about 150°C and about 225°C, more preferably about 180°C, for about one half to about 4 hours.

The invention is based on the discovery that the presence of the Bronsted acid catalyst in the reaction mixture accelerates the reaction rate and improves the yield significantly so that essentially complete (95—100%) conversion of the starting reactants occurs in about 2.5 to 3.5 hours. As a result there is made possible high efficiency in raw material use and relatively short reaction times. The high yield obtained is of advantage in

subsequent isolation and/or purification of the product salicylamide.

In terms of prior art, the effect of acid additives on amidation is controversial. For example in the amidation of unsubstituted phenyl salicylate, evidence is contradictory. Menger, F. and Smith, J. H. J. Amer Chem Soc, *91* 5346 (1969) indicate that n-butylamine hydrochloride retards the rate of aminolysis by 20%. Satchell, D. P. and Secemski, I. J. Chem Soc, B 130 (1969) find acid gives no acceleration in acetonitrile but 64—88% increase in ether. Thus the effect of acids in the reaction scheme would be difficult to predict, see e.g. Khan, M. N., J Org Chem, *48* 2046 (1983). Alternatively, US Patent No. 4 201 715 teaches the anilidisation of carboxylic acid esters using equimolar amounts of magnesium dianilide or aluminium trianilide. This method uses stoichiometric quantities of expensive reagents and results in waste disposal problems. In addition, these reagents may not be compatible with phenolic hydroxyl groups present in salicylic esters.

The mole ratio of the amine to ester is preferably about 1 to 1 to 1.5 to 1 and more preferably about 1.2 to 1 to 1.3 to 1. The mole ratio of the amine to ester selected is based mainly on economics. The most expensive reactant is the salicylate ester and, accordingly, it is desired to use a sufficient excess of the amine reactant to react completely with the ester. When $R_1$ is $—COC_nH_{2n+1}$, however, large excesses of the amine reactant are undesirable because of Schiff base side product formation by reaction of the amine with the carbonyl group of the side chain. The discovery of this side product formation makes it desirable to reflux the acidified reaction mixture in order to hydrolyse any Schiff base present and thereby recover additional desired product.

The Lowry-Bronsted acid catalyst must be strong enough to be substantially ionised and may be from a variety of sources such as aliphatic or aromatic carboxylic acids or a mineral acid such as hydrogen halides, sulfuric acid or phosphoric acid. It is also particularly convenient to utilise the hydrochloride or other hydrogen halide salt of an organic amine as such a catalyst. In its most convenient aspect, the hydrochloride salt of the reactant amine $NH_2—X—R_2$ is used. This is convenient not only for ease of handling but also because excess amine reactant is conveniently removed as the hydrochloride salt during the work-up and isolation of the product salicylamide. Thus another source of impurities is not introduced.

When the Lowry-Bronsted acid catalyst takes the form of the hydrochloride salt of the reactant amine $NH_2—X—R_2$ the amount of the free reactant amine is reduced proportionately.

After the reaction, the product salicylamide compound is recovered by any suitable method. According to one procedure described in our concurrently filed European application No. (84308164.7); publication No. 0143628, which constitutes prior art according to Art. 54(3) EPC, the reaction mixture is dissolved in a polar organic solvent (e.g. ethanol), acidified with a Lowry-Bronsted acid (e.g. hydrochloric acid) and refluxed. The product salicylamide compound is precipitated therefrom by the addition of water. The precipitated product salicylamide compound is thereafter recovered using any standard separation technique, e.g. filtration.

The polar organic solvent has a boiling point up to about 200°C and may be an alkanol, halogenated hydrocarbon or a mixture of these. The reflux step is done at a sufficiently high temperature and for a sufficient time to form the product. The temperature can vary widely depending on the solvent used, e.g. methanol, ethanol, isopropanol, n-propanol and the like. When ethanol is used, the mixture refluxes at about 80°C. It is possible to use temperatures between about 40°C and 120°C or even higher if the reflux were operated under pressure. The time of reflux may vary from a few minutes up to an hour or even longer but too lengthy a reflux could result in undesirable hydrolysis of the product. Preferably, reflux will be from about 15 to 30 minutes to minimise undesirable hydrolysis. The lower the temperature the longer the reflux heating time required while higher temperatures require shorter heating times compared to the fifteen minutes preferably used when the temperature is 80°C.

The product salicylamide compound may, if desired, be purified by recrystallisation from, for example, ethanol or an ethanol/water mixture. Any other suitable solvent in which the product salicylamide dissolves may be gainfully employed for the foregoing purpose.

The reaction between the phenyl salicylate ester and the amine $NH_2—X—R_2$ may be conducted in the presence of an inert solvent system such as a halogenated or unhalogenated aromatic compound or a polyethylene glycol of average molecular weight about 1000 to about 6000 or mixtures thereof all having a melting point of up to 120°C.

Examples of heterocyclic amines of the formula $NH_2—X—R_2$ which may be used as a reactant in the process of this invention are 2-aminofuran, 2-aminothiazole, 2-aminobenzothiazole and 8-aminopurine. The group X may be comprised of other monocyclic or fused ring polycyclic or nonfused ring polycyclic heterocyclic aromatic groups if desired.

In a preferred aspect the process of the invention is applied to the preparation of salicylamides of the formula:

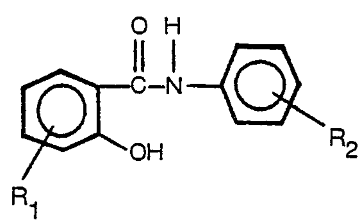

where $R_1$ and $R_2$ have the above meanings.

The following Examples, which are submitted for illustrative purposes only, demonstrate the synthesis of a representative salicylamide compound, i.e. 3′-trifluoromethyl-5-octanoylsalicylanilide from 5-octanoylphenylsalicylate and meta-trifluoromethyl aniline (1-amino-3-trifluoromethylbenzene) with remarkably high reaction rates and good effective yields.

Comparative Example

5-octanoyl phenyl salicylate is purified by repeated recrystallisation from ethanol to a constant melting point, 73—74°C. No impurities therein are observed in nuclear magnetic resonance (NMR) or infrared (IR) spectra. The purified ester (one mole) is placed in a flask with a mechanical stirrer, thermometer, condenser, rubber septum for withdrawing samples and a nitrogen purge or blanket. After meta-trifluoromethylaniline (1.2 moles) is introduced into the flask, the resulting reaction mixture is heated to 180°C. The progress of the reaction is followed by periodically withdrawing samples which are analysed by nuclear magnetic resonance spectroscopy. The product yields are calculated from the relative amount of the reactant ester and the product salicylamide.

In the above, uncatalysed reaction involving meta-trifluoromethylaniline and recrystallised 5-octanoyl phenyl salicylate (as reactants) to produce 5-octanoyl-3′-trifluoromethyl salicylanilide (OTS) the following reaction profile is observed.

| Time (Min.) | % Yield OTS |
|---|---|
| 5 | 12 |
| 30 | 27 |
| 60 | 36 |
| 90 | 48 |
| 125 | 61 |
| 160 | 65 |
| 200 | 69 |
| 235 | 71 |

Reaction times exceeding 235 min do not result in significantly greater yields, since the yield levels off after 200 min as shown above.

In this Comparative Example, and in the succeeding Examples, 0 min represents the time at which the reaction mixture reaches the temperature of 180°C.

Example 1

To the standard reaction mixture of the Comparative Example was added 10 mole per cent octanoic acid (based on the reactant ester). The progress of the reaction was as shown below in two separate runs:

RUN 1

| Time (Min.) | % Yield OTS |
|---|---|
| 0 | 17 |
| 28 | 57 |
| 64 | 76 |
| 121 | 89 |
| 176 | 100 |

RUN 2

| Time (Min.) | % Yield OTS |
|---|---|
| 0 | 24 |
| 33 | 57 |
| 60 | 68 |
| 90 | 78 |
| 106 | 79 |
| 165 | 86 |

Example 2

To the standard reaction mixture of the Comparative Example was added 10 mole per cent 5-octanoylsalicyclic acid (based on the reactant ester). The progress of the reaction was as shown below:

| Time (Min.) | % Yield OTS |
|---|---|
| 0 | 18 |
| 40 | 45 |
| 70 | 63 |
| 120 | 74 |
| 150 | 81 |
| 180 | 83 |
| 235 | 85 |

Example 3

The hydrochloride salt of meta-trifluoromethylaniline is prepared from an equal molar amount of concentrated hydrochloric acid and said aniline and it is used without further purification. An amount equal to 10 mole per cent (based on the reactant ester) was added to the reaction mixture of the Comparative Example. The progress of the reaction was as shown below:

| Time (Min.) | % Yield OTS |
|---|---|
| 0 | 20 |
| 32 | 45 |
| 60 | 60 |
| 92 | 68 |
| 121 | 73 |
| 152 | 81 |
| 180 | 100 |

Example 4

Phenyl 5-octanoyl salicylate, 20 g, and meta-trifluoromethylaniline, 12 g, were placed in a 200 ml, 3-necked round bottom flask equipped with a stirrer. Octanoic acid, 0.85 g, corresponding to 10 mole per cent (based on the salicylate reactant), was added. A nitrogen sparge was introduced and the stirred reaction mixture heated to 185°C. The reaction was monitored via NMR analysis of samples of the reaction mixture with time. After 3 hours at 185°C, conversion of the salicylate was complete. Methanol, 60 ml, was added and the solution was heated to reflux. Next, 3.5 g of 38% hydrochloric acid and 3.5 g of water was added. The acidified solution was refluxed and stirred until a precipitate formed. The reaction mixture, after allowing to stand overnight, was filtered to yield 20 g of crude product. The crude product was purified by trituration with petroleum ether followed by recrystallisation from methanol containing 1 g of concentrated hydrochloric acid. The purified product, 3′-trifluoromethyl-5-octanoylsalicylanilide, amounted to 17 g of 71% of theory.

**Claims**

1. A process for preparing a salicylamide compound of the formula:

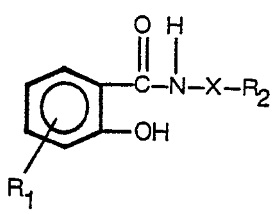

where

X is a benzene ring or a heterocyclic aromatic group

$R_1$ is a hydrogen atom or a substituent group which is $-COC_nH_{2n+1}$ or $-C_nH_{2n+1}$ where n is an integer of from 1 to 15, and

$R_2$ is a hydrogen atom or a substituent group which is CN, $NO_2$, F, Cl, Br, I, $CF_3$, $CCl_3$, $CBr_3$, $Cl_3$, $COC_nH_{2n+1}$ or $C_nH_{2n+1}$

which process comprises reacting together one mole part of a phenyl salicylate ester optionally bearing an $R_1$ substituent on the benzene ring of the salicylic acid portion thereof with one to two mole parts of an amine $NH_2-X-R_2$ and with 0.01 to 0.25 mole part of a Lowry-Bronsted acid catalyst at a temperature of above 150°C for one half to 4 hours.

2. A process as claimed in Claim 1 wherein the phenyl salicylate ester and the amine are reacted at a temperature in the range of 150°C to 225°C.

3. A process as claimed in Claim 2 wherein the temperature is 180°C.

4. A process as claimed in any of Claims 1 to 3 wherein the Lowry-Bronsted acid catalyst is an aliphatic carboxylic acid, an aromatic carboxylic acid, a hydrogen halide, a hydrogen halide salt of an organic amine or a mixture thereof.

5. A process as claimed in Claim 4 wherein the Lowry-Bronsted acid catalyst is the hydrochloride salt of the amine $NH_2-X-R_2$.

6. A process as claimed in Claim 5 which comprises reacting together one mole part of the optionally substituted phenyl salicylate ester with 1.1 mole parts of the amine and with 0.1 mole part of the hydrochloride salt of the amine.

7. A process as claimed in Claim 4 wherein the Lowry-Bronsted acid catalyst is octanoic acid, 5-octanoylsalicylic acid or meta-trifluoromethylaniline hydrochloride.

8. A process as claimed in any of Claims 1 to 5 wherein the quantity of the Lowry-Bronsted acid is 0.1 mole part.

9. A process as claimed in any of the preceding claims wherein the phenyl salicylate ester, the amine and the Lowry-Bronsted acid catalyst are reacted in the presence of an organic solvent system consisting of a halogenated aromatic compound having a melting point of up to 120°C, an unhalogenated aromatic compound having a melting point of up to 120°C, a polyethylene glycol having an average molecular weight of 1000 to 6000 or a mixture thereof.

10. A process as claimed in any of Claims 1 to 9 wherein the amine $NH_2-X-R_2$ is aniline or an $R_2$-substituted aniline.

**Patentansprüche**

1. Verfahren zur Herstellung einer Salicylamidverbindung der Formel:

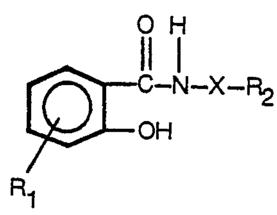

worin

X ein Benzolring oder eine heterozyklische aromatische Gruppe ist,

$R_1$ ein Wasserstoffatom oder eine Substituentengruppe ist, die $-COC_nH_{2n+1}$ oder $-C_nH_{2n+1}$ ist, worin n eine ganze Zahl von 1 bis 15 ist, und

$R_2$ ein Wasserstoffatom oder eine Substituentengruppe ist, die CN, $NO_2$, F, Cl, Br, I, $CF_3$, $CCl_3$, $CBr_3$, $Cl_3$, $COC_nH_{2n+1}$ oder $C_nH_{2n+1}$ ist, wobei das Verfahren das gemeinsame Umsetzen von einem Mol-Teil eines Phenylsalicylatesters, der gegebenenfalls einen $R_1$-Substituenten an dem Benzolring des Salicylsäureteiles davon aufweist, mit einem bis zwei Mol-Teilen eines Amins $NH_2X-R_2$ und mit 0,01 bis 0,25 Mol-Teilen eines Lowry-Brönsted Säurekatalysators bei einer Temperatur von über 150°C für eine halbe bis 4 Stunden umfaßt.

2. Verfahren nach Anspruch 1, worin der Phenylsalicylatester und das Amin bei einer Temperatur in dem Bereich von 150°C bis 225°C umgesetzt werden.

3. Verfahren nach Anspruch 2, worin die Temperatur 180°C beträgt.

4. Verfahren nach einem der Ansprüche 1 bis 3, worin der Lowry-Brönsted Säurekatalysator eine aliphatische Carbonsäure, eine aromatische Carbonsäure, ein Wasserstoffhalogenid, ein Wasserstoffhalogenidsalz eines organischen Amins oder eine Mischung davon ist.

5. Verfahren nach Anspruch 4, worin der Lowry-Brönsted Säurekatalysator das Hydrochloridsalz des Amins $NH_2—X—R_2$ ist.

6. Verfahren nach Anspruch 5, das das gemeinsame Umsetzen von einem Mol-Teil des gegebenenfalls substituierten Phenylsalicylatesters mit 1,1 Mol-Teilen des Amins und mit 0,1 Mol-Teil des Hydrochloridsalzes des Amins umfaßt.

7. Verfahren nach Anspruch 4, worin der Lowry-Brönsted Säurekatalysator Octylsäure, 5-Octanoylsalicylsäure oder meta-Trifluormethylanilinhydrochlorid ist.

8. Verfahren nach einem der Ansprüche 1 bis 5, worin die Menge der Lowry-Brönsted Säure 0,1 Mol-Teil beträgt.

9. Verfahren nach einem der vorhergehenden Ansprüche, worin der Phenylsalicylatester, das Amin und der Lowry-Brönsted Säurekatalysator in Gegenwart eines organischen Lösungsmittelsystems bestehend aus einer halogenierten aromatischen Verbindung mit einem Schmelzpunkt von bis zu 120°C, einer nichthalogenierten aromatischen Verbindung mit einem Schmelzpunkt von bis zu 120°C, einem Polyethylenglykol mit einem durchschnittlichen Molekulargewicht von 1000 bis 6000 oder einer Mischung davon umgesetzt wird.

10. Verfahren nach einem der Ansprüche 1 bis 9, worin das Amin $NH_2—X—R_2$ Anilin oder ein $R_2$-substituiertes Anilin ist.

**Revendications**

1. Procédé de préparation d'un composé salicylamide de formule:

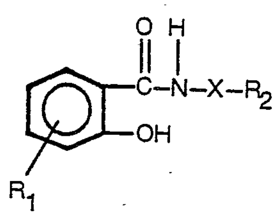

dans laquelle

X est un noyau benzénique ou un groupe aromatique hétérocyclique,

$R_1$ est un atome d'hydrogène ou un groupe substituant qui est $—COC_nH_{2n+1}$ ou $—C_nH_{2n+1}$, $n$ étant un nombre entier de 1 à 15 et

$R_2$ est un atome d'hydrogène ou un groupe substituant qui est CN, $NO_2$, F, Cl, Br, I, $CF_3$, $CCl_3$, $CBr_3$, $Cl_3$, $COC_nH_{2n+1}$ ou $C_nH_{2n+1}$,

procédé qui consiste à faire réagir ensemble 1 mole partie d'un salicylate de phényle portant facultativement un substituant $R_1$ sur le noyau benzène de la portion acide salicylique avec 1 à 2 moles partie d'une amine $NH_2—X—R_2$ et avec 0,01 à 0,25 mole partie d'un catalyseur acide Lowry-Bronsted à une température supérieure à 150°C pendant 0,5 à 4 heures.

2. Procédé selon la revendication 1, dans lequel on fait réagir le salicylate de phényle et l'amine à une température comprise entre 150 et 225°C.

3. Procédé selon la revendication 2, dans lequel la température est de 180°C.

4. Procédé selon l'une quelconque des revendications 1 à 3, dans lequel le catalyseur acide Lowry-Bronsted est un acide carboxylique aliphatique, un acide carboxylique aromatique, un acide halogénhydrique, un halogènhydrate d'une amine organique ou un mélange de ceux-ci.

5. Procédé selon la revendication 4, dans lequel le catalyseur acide Lowry-Bronsted est le chlorhydrate de l'amine $NH_2—X—R_2$.

6. Procédé selon la revendication 5, qui comprend l'étape consistant à faire réagir ensemble 1 mole partie du salicylate de phényle facultativement substitué avec 1,1 mole partie de l'amine et avec 0,1 mole partie du chlorhydrate de l'amine.

7. Procédé selon la revendication 4, dans lequel le catalyseur acide Lowry-Bronsted est l'acide octanoïque, l'acide 5-octanoyl-salicylique ou le chlorhydrate de m-trifluorométhylaniline.

8. Procédé selon l'une quelconque des revendications 1 à 5, dans lequel la quantité de l'acide Lowry-Bronsted est de 0,1 mole partie.

9. Procédé selon l'une quelconque des revendications précédentes, dans lequel on fait réagir le salicylate de phényle, l'amine et le catalyseur acide Lowry-Bronsted en présence d'un système solvant organique comprenant un composé aromatique halogéné ayant un point de fusion pouvant aller jusqu'à 120°C, un composé aromatique non halogéné ayant un point de fusion pouvant aller jusqu'à 120°C, un polyéthylèneglycol ayant une masse moléculaire moyenne de 1000 à 6000 ou un mélange de ceux-ci.

10. Procédé selon l'une quelconque des revendications 1 à 9, dans lequel l'amine $NH_2—X—R_2$ est l'aniline ou une aniline substituée par $R_2$.